Europäisches Patentamt

European Patent Office

Office européen des brevets

(19)

(11) Publication number: **0 165 797**
**A2**

(12) # EUROPEAN PATENT APPLICATION

(21) Application number: **85304314.9**

(22) Date of filing: **17.06.85**

(51) Int. Cl.⁴: **C 07 C 141/04**

(30) Priority: **18.06.84 GB 8415492**

(43) Date of publication of application:
**27.12.85 Bulletin 85/52**

(84) Designated Contracting States:
**DE FR GB IT**

(71) Applicant: **UNILEVER PLC**
**Unilever House Blackfriars P.O. Box 68**
**London EC4P 4BQ(GB)**

(84) Designated Contracting States:
**GB**

(71) Applicant: **UNILEVER NV**
**Burgemeester s'Jacobplein 1 P.O. Box 760**
**NL-3000 DK Rotterdam(NL)**

(84) Designated Contracting States:
**DE FR IT**

(72) Inventor: **Fairclough, Colin Sydney**
**Quarry NY Brancote Gardens Bromborough**
**Wirral Merseyside L62 6AH(GB)**

(72) Inventor: **Littler, Jane Annie**
**The New Pale Manley**
**Warrington Cheshire WA6 9EY(GB)**

(74) Representative: **Mole, Peter Geoffrey et al,**
**UNILEVER PLC Patents Division P.O. Box 68 Unilever**
**House**
**London EC4P 4BQ(GB)**

(54) Process for the manufacture of alcohol sulphate.

(57) A process for manufacturing pastes of sodium alkyl sulphates of relatively high concentration from alkyl sulphuric acid is disclosed. The process involves addition of sodium ion in excess of that required to neutralise the free acid. The process is particularly applicable to neutralisation of alkyl sulphuric acid derived from coconut or palm kernel oil.

EP 0 165 797 A2

PROCESS FOR THE MANUFACTURE OF ALCOHOL SULPHATE

This invention relates to the manufacture of concentrated solutions or suspensions of primary alkyl sulphates.

Primary alkyl sulphates, particularly the sodium, potassium, ammonium and substituted ammonium salts are well known detergent-active compounds finding use in fabric washing powders and bars, in shampoos and in toothpastes. In some countries, chiefly the southern and eastern ones, primary alkyl sulphates are much more commercially attractive than alkyl benzene sulphonates because of the plentiful supply of primary alcohols in the form of coconut oil and other naturally occurring oils which can serve as a starting material for their manufacture. We are seeking ways to improve our use of these naturally derived detergents.

Alkyl sulphates are normally manufactured by sulphation of alcohols with an $SO_3$/air mixture using a falling-film reactor. In this type of reactor the $SO_3$/air mixture is passed co-currently over a falling thin film of alcohol feedstock formed on a cooled surface. In this way the heat produced during the reaction can be quickly extracted and discolouration avoided. Under these conditions a reaction such as a sulphation goes substantially to completion, so at the bottom of the film, the liquid consists substantially of alkyl sulphuric acid. This is then passed to a neutralisation loop. That is to say it is pumped into a circular system into which caustic soda solution is pumped in the opposite direction, a solution or slurry of the sodium salt of the alkyl sulphate being removed. The concentration of the sodium hydroxide which is pumped into the neutralisation loop is critical in determining the concentration of the alkyl sulphate which emerges, since apart from the water produced during the neutralisation reaction, the water introduced with the sodium hydroxide is the only water in the system. Up until now it has only been possible to produce alkyl sulphate pastes of concentrations up to about 40-45% by weight by means of the technique just described. It may not matter if the alkyl sulphate paste is relatively dilute when it is intended to be used for making a crutcher slurry for spray-drying, since a large quantity of water will necessarily be present anyway, but when the alkyl sulphate is intended for incorporation into a detergent bar, then any excess water has to be evaporated, which is wasteful of energy. For maximum flexibility, therefore, as concentrated a solution as possible should be prepared, which means introducing as little water as possible with the sodium hydroxide.

The relationship between concentration and viscosity of solutions and suspensions of primary alkyl sulphates is complex. Considering the situation at constant temperature and shear rate, as the concentration is increased the viscosity of the solution generally increases, but at a concentration of about 45% by weight a steep increase in viscosity is experienced at the temperatures encountered in neutralisation loops. In the past this viscosity increase problem has led manufacturers to take special measures to produce material which is sufficiently mobile, since it can have implications for the mechanical design of pumps, mixers, pipe sizing and equipment layout. Two such measures are the introduction of co-surfactants, and adherence to cations other than alkali-metal cations.

We have now discovered how to manufacture pastes of alkyl sulphate salts containing 55-80% by weight or more of alkyl sulphate which remain mobile and pumpable. The technique exploits a viscosity minimum which occurs at such concentrations.

Accordingly, the present invention provides a process for the manufacture of a paste of a sodium primary alkyl hydrogen sulphate substantially free from co-surfactants which comprises:

(a) sulphating a primary alcohol feedstock with a gaseous mixture comprising sulphur trioxide and oxygen to produce a reaction product containing an alkyl sulphuric acid;

(b) neutralising the alkyl sulphuric acid with an equivalent of aqueous sodium hydroxide of a concentration such that a paste of the alkyl hydrogen sulphate salt is produced having a concentration of at least 55% by weight;

(c) substantially simultaneously adding from ½ to 5% by weight of a sodium ion to the paste thereby reducing its viscosity.

As indicated above it has already been proposed that pastes of alkyl sulphates having relatively high concentrations should be manufactured. However, it has been necessary to adopt the use of expensive organic counter-ions in order to reach the concentration range which we specify. For example, British patent No 1 488 352 (Albright & Wilson Limited) describes how difficult it is to produce pastes containing more than about 30-45% by weight, even with the use of viscosity modifiers. But it is said that if the alkyl sulphuric acid is reacted with ammonia or with an organic amine containing from 1 to 6 carbon atoms, progress can be made in reducing viscosity.

British patent No 1 538 032 (Kao Soap) describes the manufacture of aqueous solutions or pastes of high alkyl sulphates or ethoxylated high alkyl sulphates containing from 25 to 50% by weight of the sulphate. Special measures are taken to achieve these concentrations, namely the addition of sulphuric acid and an alkali metal sulphate to the unneutralised alkyl sulphuric acid, but they are not as high as the concentrations which we are able to achieve in our process.

Lastly, British patent No 2 029 141 (Albright & Wilson Limited) discloses manufacture of pastes having a concentration in the range which we specify, but it is essential for at least 5% by weight of an additional class of surfactant to be present during the neutralisation, in addition to the alkyl sulphuric acid, whereas our process enables an alkyl sulphuric acid salt paste to be manufactured in the absence of co-surfactants.

The concentration of sodium hydroxide needed, for example, to neutralise the alkyl sulphuric acid to produce a paste having a concentration of 60% by weight or more is 18% by weight.    Additionally, a level of excess sodium ion of up to 1.7% by weight will be required to control the viscosity, so if, as is preferred, this excess sodium ion is supplied by the sodium hydroxide, then an additional 3% or so by weight of the hydroxide will be needed.

Sodium chloride and sodium sulphate are also suitable sources of excess sodium ion.

The process of the invention is especially suitable for making coconut and palm kernel oil alkyl sulphates, which have carbon chain lengths in the $C_{12}-C_{14}$ region.

Care must be taken with the rate of addition of sodium hydroxide to the neutralisation loop, since any local areas of abnormal composition can lead to excessive paste thickening at pH's above 12 and to product degradation at pH's below 7.

Our process is further described in the following example:

Example

A feedstock of a natural alcohol (Picol 2455, registered trade mark, manufactured and sold by Kao Soap Company) was sulphated on a falling film reactor manufactured by Allied Chemical Company under the following conditions:

| Alcohol feed rate | : | 12 Kg/hr |
| Feed temperature | : | 35°C |
| $SO_3$ feed rate | : | 5.6 Kg/hr |
| Mole ratio $SO_3$:alcohol | : | 1.15 |
| Diluent air rate | : | 495 litres/min |
| Acid transfer time to neutraliser | : | 60 sec |
| $SO_3$ concentration in air | : | 5% v/v |

The alkyl sulphuric acid produced in this reactor was then neutralised in a neutralisation loop under the conditions shown in the table, in which feed rates are in kg/hr, and all percentages are expressed by weight, and viscosities are expressed in poise.

TABLE

| Example | Alkyl Sulphuric Acid Feed Rate | Sodium Hydroxide Concn. | Sodium Hydroxide Feed Rate | Additional Na$^+$ ion Source | Additional Na$^+$ ion Concn. | Additional Na$^+$ ion Feed Rate | % Active Paste | Concn. Na$^+$ as % Active | Viscosity (poise) |
|---|---|---|---|---|---|---|---|---|---|
| 1 | 100 | 38.8 | 37.7 | NaOH | Same | Same | 78 | 0.4 | 60 |
| 2 | 100 | 30.3 | 58.0 | NaOH | Same | Same | 70 | 2.0 | ∼20 |
| 3 | 100 | 37.5 | 37.8 | Na$_2$SO$_4$ | 27 | 16 | 70 | 4.0 | 45 |
| 4 | 100 | 37.5 | 37.8 | NaCl | 27 | 16 | 70 | 4.0 | 25 |
| 5 | 100 | 24.0 | 86.0 | NaOH | Same | Same | 58 | 3.0 | 170 |
| 6 | 100 | 24.0 | 86.0 | NaOH | Same | Same | 58 | 6.0 | 80 |

Note: The term "% Active Paste" refers to the percentage of sodium alkyl sulphate in the neutralised paste. The term "Concn. Na$^+$ as % Active" refers to the percentage of sdoium, sodium sulphate or sodium chloride present as a percentage of the total weight of sodium alkyl sulphate. In the case of sodium hydroxide it is the additional percentage present over and above the amount needed completely to neutralise the alkyl sulphuric acid.

C.3041

It can be seen that in comparison with the viscosities which would have been exhibited by pastes of these concentrations in the absence of additional sodium ion ($\sim$1000 poise), the pastes are extremely mobile.

## CLAIMS:

1. A process for the manufacture of a paste of a sodium primary alkyl hydrogen sulphate which comprises:

(a) sulphating a primary alcohol feedstock with a gaseous mixture comprising sulphur trioxide and oxygen to produce a reaction product containing an alkyl sulphuric acid;

(b) neutralising the alkyl sulphuric acid with an equivalent of aqueous sodium hydroxide of a concentration such that a paste of the alkyl hydrogen sulphate salt is produced having a concentration of at least 55% by weight;

(c) substantially simultaneously adding from ½ to 5% by weight of sodium ion to the paste thereby reducing its viscosity.

2. A process according to claim 1 or claim 2 wherein the sodium ion is added in the form of a highly dissociated salt.

3. A process according to claim 3 wherein the highly dissociated salt is sodium sulphate or sodium chloride.

4. A process according to any one of the preceding claims wherein the sodium ion is added in the form of sodium hydroxide in excess of the amount required for neutralisation.

5. A process according to any one of the preceding claims wherein the alcohol feedstock is derived from coconut oil.

C.304 0165797

6.   A process according to any one of the preceding claims wherein the neutralisation of the alkyl sulphuric acid takes place at a temperature of from 40° to 75°C.

7.   A process according to claim 5 wherein the neutralisation takes place at a temperature of at least 50°C.